Europäisches Patentamt

European Patent Office

Office européen des brevets

(19)

(11) Numéro de publication: **0 437 710 A1**

# DEMANDE DE BREVET EUROPEEN

(12)

(21) Numéro de dépôt: 90122936.9

(22) Date de dépôt: 30.11.90

(51) Int. Cl.5: **C12P 7/64**

(30) Priorité: 17.01.90 CH 146/90

(43) Date de publication de la demande:
24.07.91 Bulletin 91/30

(84) Etats contractants désignés:
AT BE CH DE DK ES FR GB GR IT LI LU NL SE

(71) Demandeur: SOCIETE DES PRODUITS NESTLE S.A.
Case postale 353
CH-1800 Vevey(CH)

(72) Inventeur: Borel, Christian
Route d'Echallens 5
CH-1032 Romanel(CH)
Inventeur: Hansen, Carl Erik
Ch. du Bois Murat, 20
CH-1066 Epalinges(CH)

(54) Procédé de production d'acides gras polyinsaturés.

(57) Afin de produire des acides gras polyinsaturés, on cultive des cellules d'une mousse choisie dans un groupe formé par les espèces Eurhynchium striatum, Brachythecium rutabulum, Brachythecium salebrosum, Rhytidiadelphus squarrosus, Scleropodium purum et Rhytidiadelphus triquetrus, et l'on en extrait les composés recherchés.

EP 0 437 710 A1

## PROCÉDÉ DE PRODUCTION D'ACIDES GRAS POLYINSATURÉS

La présente invention a trait à un procédé de production d'acides gras polyinsaturés, tels que par exemple l'acide arachidonique ou l'acide eicosapentaenoïque.

Il est fait, actuellement, une utilisation croissante des acides gras polyinsaturés dans l'industrie alimentaire ou cosmétique, due particulièrement au fait de leurs propriétés remarquables.

L'acide arachidonique, par exemple, joue un rôle important en tant que précurseur des leucotriènes et des prostaglandines, éléments essentiels pour la régulation de certaines fonctions cellulaires et physiologiques. L'acide eicosapentaenoïque, quant à lui, semble jouer un rôle important dans la prévention des risques de thrombose ou d'artériosclérose.

Il est connu, par exemple par le brevet EP 276982, de produire des acides gras polyinsaturés par réaction enzymatique à l'aide d'un microorganisme du type Mortierella, ou bien par culture de ce même microorganisme en présence d'un hydrocarbone et d'un acide gras, ainsi que le décrit le brevet EP 276541. Un autre procédé de production d'acides gras insaturés, décrit dans le brevet EP 304049, consiste à cultiver un microorganisme du type Conidiobolus sur un milieu contenant un acide gras insaturé comme source de carbone. Encore un autre procédé, décrit dans le brevet EP 277747, consiste à produire de l'acide eicosapentaenoïque à partir d'algues appartenant au type Corallina.

La présente invention a pour but de proposer un procédé permettant de produire, d'une manière simple, et en quantité importante, des acides gras polyinsaturés d'origine naturelle, à savoir extraits de mousses.

A cet effet, le présent procédé est caractérisé par le fait que l'on cultive des cellules d'une mousse choisie dans un groupe formé par les espèces Eurhynchium striatum, Brachythecium rutabulum, Brachythecium salebrosum, Rhytidiadelphus squarrosus, Scleropodium purum et Rhytidiadelphus triquetrus et que l'on en extrait les acides gras polyinsaturés recherchés.

Un avantage de la présente invention est de permettre d'obtenir une culture stabilisée de cellules des mousses sélectionnées, ladite culture produisant d'une manière constante et reproductible, les acides gras polyinsaturés recherchés.

Un autre avantage de la présente invention est de permettre la production d'acides gras insaturés généralement liés à des glycolipides particulièrement indiqués pour une utilisation dans les produits cosmétiques.

Les mousses sont principalement constituées de deux parties: une phase haploïde ou gamétophyte et une phase diploïde ou sporophyte.

Des spores haploïdes sont produites par le sporophyte après la méïose. Ces spores peuvent se développer, par germination, afin de donner le gaméthophyte.

Afin de mettre en oeuvre le procédé selon la présente invention, on peut collecter les capsules renfermant lesdites spores, de préférence lorsqu'elles sont en phase de maturation.

Les capsules collectées peuvent être stérilisées, par exemple par trempage dans une solution diluée de chlorure de sodium, suivi d'un trempage rapide dans l'éthanol et d'un rinçage à l'eau distillée, ou par tout autre moyen permettant d'éliminer les éventuels germes présents à leur surface.

Les capsules stérilisées peuvent être ouvertes et les spores qu'elles contiennent disposées sur un milieu de culture usuel dans le domaine de la culture de spores, tel que, par exemple, un milieu de Murashige et Skoog (Murashige T. et Skoog F. (1962), Physiol. Plant. 15, 473-497), de préférence dilué à 8-12% en poids. On peut utiliser, de préférence, un milieu de culture sous forme semi-solide, contenant par exemple 1-2% en poids d'agar, et exempt d'hormones de croissance et de vitamines.

Les spores peuvent être cultivées sur ce milieu, de préférence à une température de 15-30°C et sous un éclairement de 2000-7000 lux pendant 14-18 heures suivi d'une période d'obscurité de 6-10 heures, pendant 1 à 4 mois, afin de se développer en cellules.

On peut alors récupérer les cellules ainsi obtenues et les inoculer dans un milieu de culture liquide tel que, par exemple, un milieu de Murashige et Skoog. Ce milieu peut être utilisé tel quel, afin de permettre d'augmenter la fraction lipidique totale, ou dilué à 8-12% en poids, afin d'améliorer l'accumulation de biomasse. Le milieu de culture peut être, de préférence, exempt d'hormones de croissance et de vitamines, mais peut contenir 0 à 25 g de glucose par litre de milieu de culture.

On peut inoculer, par exemple, 0,1-0,8 mg de matière sèche de cellule par ml de milieu de culture.

On peut ajuster le pH du milieu de culture liquide à une valeur de l'ordre de 5,5-7,0, par exemple par addition d'une solution de tampon biologique usuel dans le domaine de la culture de tissus.

Cette stabilisation du pH peut permettre d'obtenir une différentiation rapide des cellules et une production de biomasse plus importante.

On peut ajouter, par exemple, de l'acide 2-morpholinoéthanesulfonique à 0,05-0,2% en poids.

On peut laisser incuber les cellules pendant 2 à 6 semaines, à une température de 15-30° C et sous un éclairement de 2000-7000 lux pendant environ 14-18h suivi de 6-10h d'obscurité.

On peut agiter le milieu pendant tout ou partie de l'incubation, par exemple avec un agitateur rotatif tournant à 100-120 tours par minute.

Après incubation, on peut récolter les cellules, par exemple par filtration ou par centrifugation.

On peut ensuite homogénéiser les cellules récoltées, par exemple dans une solution contenant 1-3 parties en poids de chloroforme pour une partie en poids de méthanol, dans une solution d'hexane ou dans une solution d'isopropanol ou dans un mélange de ces composés. Préalablement à l'extraction, on peut tremper les cellules pendant 2 à 6 minutes dans une solution bouillante d'isopropanol, afin d'inhiber l'activité des lipases.

L'extrait obtenu peut être clarifié par filtration puis séché, par exemple dans un évaporateur rotatif à 35 -40° C, sous pression réduite.

La fraction lipidique ainsi obtenue contient les acides gras polyinsaturés recherchés, ainsi que d'autres acides gras.

On peut séparer les différents acides gras polyinsaturés contenus dans la fraction lipidique.

On peut, par exemple, faire une transestérification à l'aide de chlorure d'acétyle, afin d'obtenir les esters méthyliques des acides gras polyinsaturés recherchés, puis extraire ces esters, par exemple avec de l'hexane. Les esters ainsi obtenus peuvent être identifiés, par exemple par chromatographie en phase gazeuse, par comparaison avec les caractéristiques connues des composés standards, ou par spectrométrie de masse.

Les acides gras polyinsaturés ainsi obtenus peuvent être utilisés dans les produits alimentaires et/ou cosmétiques.

La présente invention est illustrée plus en détail dans les exemples ci-après.

## Exemple 1

On inocule 100 mg de matière sèche de cellules de Rhytidiadelphus squarrosus dans 200 ml de milieu de culture liquide de Murashige et Skoog dilué à 10% en poids, exempt d'hormones de croissance et de vitamines et contenant 10 g de glucose par litre.

On ajuste le pH du milieu de culture à 6,5 par addition d'acide 2-morpholinoéthanesulfonique à 0,1% en poids. On laisse les cellules incuber à 20° C, sous un éclairement de 5000 lux pendant 16 heures suivi de 8 heures d'obscurité, sous agitation constante à 110 tours par minute.

Après 3 semaines d'incubation, on filtre la suspension et l'on obtient 820 mg de matière sèche de cellules.

On trempe les cellules obtenues dans une solution d'isopropanol à 80-90° C, pendant 2 minutes et on les en sort. On met les cellules en suspension dans une solution contenant 2 parties de chloroforme pour 1 partie de méthanol. On filtre l'extrait obtenu puis on le sèche dans un évaporateur rotatif à 40° C, sous pression réduite et on le purifie par extraction à l'aide d'une solution de KCl à 0,88% en poids. On obtient 52,5 mg de fraction lipidique, soit 6,4% en poids de la quantité de matière sèche initiale de cellules.

On transestérifie les 52,5 mg de fraction lipidique avec du chlorure d'acétyle, puis l'on extrait les esters méthyliques ainsi formés avec de l'hexane.

Les esters obtenus sont séparés par chromatographie en phase gazeuse, et identifiés par comparaison entre autre de leur spectre de masse avec les spectres de masse des composés standards.

Dans le tableau ci-après, les esters d'acides gras sont désignés par le nombre d'atomes de carbone, le nombre et la position des doubles liaisons, de l'acide gras de base.

Ledit extrait des esters méthyliques comprend la totalité des acides gras de ladite fraction lipidique sous forme estérifiée et présente la composition suivante, en % en poids:

| Acide gras | % de l'ester de l'acide considéré par rapport à la quantité totale d'acide gras estérifié |
|---|---|
| 16:0 | 17,0 |
| 16:1 | 2,2 |
| 18:0 | 0,8 |
| 18:1 | 1,6 |
| 18:2 | 16,4 |
| 18:3 (n-6) | 2,0 |
| 18:3 (n-3) | 16,7 |
| 20:3 (n-6) | 5,7 |
| 20:4 (n-6) | 32,4 |
| 20:5 (n-3) | 5,2 |

On obtient 5,8 mg d'arachidonate de méthyl (20:4, n-6) et 0,98 mg d'eicosapentaenoate de méthyl (20:5, n-3).

Exemple 2

On inocule de la même manière que dans l'exemple 1, 100 mg de matière sèche de cellules de différentes mousses dans un milieu de culture liquide de Murashige et Skoog, qui peut être dilué ou non, et dont le pH peut être ajusté à 6,5 par addition d'acide 2-morpholinoéthanesulfonique à 0,1% en poids (acide MES).

On détermine la quantité d'acide arachidonique (AA) et d'acide eicosapentaenoïque (EPA) présents, en % en poids de l'acide considéré par rapport à la quantité totale d'acide gras (I), ainsi que en mg par g de matière sèche de cellules de mousse (II).

On obtient les résultats suivants:

| mousses | I (%) | | II (mg/g) | |
|---|---|---|---|---|
| | AA | EPA | AA | EPA |
| B.rutabulum | 40,1 | 4,7 | 7,4 | 0,9 |
| B.salebrosum | 32,2 | 4,1 | 16,7 | 2,1 |
| R.triquetrus | 20,2 | 2,0 | 4,8 | 0,5 |
| R.squarrosus | 32,4 | 5,2 | 7,1 | 1,2 |
| S.purum | 41,5 | 2,6 | 16,5 | 1,0 |
| E.striatum | 33,6 | 2,7 | 7,0 | 0,6 |

Le milieu de culture liquide utilisé pour chaque mousse est:
- pour B.rutabulum, R.triquetrus et R.squarrosus, un milieu de Murashige et Skoog dilué à 10% en poids, contenant de l'acide MES (pH 6,5)

- pour B.salebrosum, un milieu de Murashige et Skoog non dilué, contenant de l'acide MES (pH 6,5)
- pour S.purum, un milieu de Murashige et Skoog non dilué (pH 5,8)
- pour E.striatum, un milieu de Murashige et Skoog dilué à 10% en poids (pH 5,8).

Exemple 3

On inocule 20 mg de matière sèche de cellules de R.squarrosus dans 40 ml de milieu de culture liquide de Murashige et Skoog, exempt d'hormones et de vitamines, et contenant 10 g/l de glucose, de pH = 5,8.
On laisse les cellules incuber à 20°C, sous un éclairement de 5000 lux pendant 16 heures suivi de 8 heures d'obscurité, sous agitation constante à 110 tours par minute.
Après 3 semaines d'incubation, on filtre la suspension et l'on obtient 30 mg de matière sèche de cellules.
On trempe les cellules obtenues dans une solution d'isopropanol à 80-90°C pendant 2 minutes et on les en sort. On met les cellules en suspension dans une solution contenant 2 parties de chloroforme pour 1 partie de méthanol.
On filtre, sèche et purifie l'extrait selon l'exemple 1 et l'on obtient 4,7 mg de fraction lipidique, c'est-à-dire 15,6% en poids de la quantité de matière sèche initiale de cellules.
On transestérifie la fraction lipidique avec du chlorure d'acétyle puis l'on extrait les esters méthyliques avec de l'hexane.
On obtient, après séparation, 0,40 mg d'arachidonate de méthyl, soit 13,2 mg/g de matière sèche de cellules et 0,06 mg d'eicosapentaenoate de méthyl, soit 2,1 mg/g de matière sèche de cellules.

**Revendications**

1. Procédé de production d'acides gras polyinsaturés, caractérisé par le fait que l'on cultive des cellules d'une mousse choisie dans un groupe formé par les espèces Eurhynchium striatum, Brachythecium rutabulum, Brachythecium salebrosum, Rhytidiadelphus squarrosus, Scleropodium purum et Rhytidiadelphus triquetrus, et que l'on en extrait les acides gras polyinsaturés recherchés.

2. Procédé selon la revendication 1, caractérisé par le fait que les cellules de mousses sont obtenues par culture de spores, à 15-30°C, sous un éclairement de 2000 -7000 lux pendant 14-18 heures suivi de 6-10 heures d'obscurité, sur un milieu de culture semi-solide pendant 1 à 4 mois.

3. Procédé selon la revendication 1, caractérisé par le fait que l'on cultive les cellules sur un milieu de culture liquide à 15-30°C, sous un éclairement de 2000-7000 lux pendant 14-18 heures suivi de 6-10 heures d'obscurité, pendant 2 à 6 semaines.

4. Procédé selon la revendication 3, caractérisé par le fait que l'on ajuste le pH du milieu de culture liquide à une valeur de 5,5-7,0.

5. Procédé selon la revendication 4, caractérisé par le fait que l'on ajuste le pH par addition d'acide 2-morpholinoéthanesulfonique à 0,05-0,2% en poids.

6. Procédé selon l'une des revendications 2 et 3, caractérisé par le fait que le milieu de culture est un milieu de Murashige et Skoog dilué à 8-12% en poids, ou non dilué.

7. Procédé selon la revendication 3, caractérisé par le fait que le milieu de culture liquide est exempt d'hormones de croissance et de vitamines, et qu'il contient 0 à 25 g/l de glucose.

8. Procédé selon la revendication 1, caractérisé par le fait que l'on extrait les acides gras polyinsaturés des cellules à l'aide de chloroforme, de méthanol, d'isopropanol, d'hexane ou de mélanges de ces composés.

9. Utilisation des acides gras polyinsaturés obtenus selon la revendication 1, dans les produits alimentaires et/ou cosmétiques.

**Office européen**
**des brevets**

# RAPPORT DE RECHERCHE
# EUROPEENNE

Numéro de la demande

# EP 90 12 2936

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | JP-A-1 243 993  (SUNTORY LTD)<br>* Résumé *<br><br>– – – | 1-9 | C 12 P<br>7/64 |
| Y | CHEMICAL ABSTRACTS, vol. 71, 1969, page 90, résumé no. 88441u, Columbus, Ohio, US; H. WAGNER et al.: "Isolation of arachidonic acid from the mosses Rhytidiadelphus triquetrus, polytrichum commune, Fegatella conica", & PHYTOCHEMISTRY 1969, 8(8), 1603<br>* Résumé *<br><br>– – – – – | 1-9 | |

**DOMAINES TECHNIQUES**
**RECHERCHES (int. Cl.5)**

C 12 P

Le présent rapport de recherche a été établi pour toutes les revendications

| Lieu de la recherche | Date d'achèvement de la recherche | Examinateur |
|---|---|---|
| La Haye | 23 avril 91 | REMPP G.L.E. |